(19) Europäisches Patentamt
European Patent Office
Office européen des brevets

(11) EP 1 518 900 A2

(12) EUROPÄISCHE PATENTANMELDUNG

(43) Veröffentlichungstag:
**30.03.2005 Patentblatt 2005/13**

(51) Int Cl.[7]: **C08L 71/02**, C08G 65/32

(21) Anmeldenummer: **04020723.5**

(22) Anmeldetag: **01.09.2004**

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(30) Priorität: **15.09.2003 DE 10342870**

(71) Anmelder: **Clariant GmbH**
**65929 Frankfurt am Main (DE)**

(72) Erfinder:
- **Löffler, Matthias, Dr.**
  **65527 Niedernhausen (DE)**
- **Klug, Peter, Dr.**
  **63762 Grossostheim (DE)**
- **Scherl, Franz Xaver, Dr.**
  **84508 Burgkirchen (DE)**

(74) Vertreter: **Mikulecky, Klaus, Dr. et al**
**Clariant Service GmbH**
**Patente, Marken, Lizenzen**
**Am Unisys-Park 1**
**65843 Sulzbach (DE)**

# (54) Flüssige Zusammensetzungen enthaltend oxalkylierte Polyglycerinester

(57) Es werden Zusammensetzungen beschrieben, die oxalkylierte Polyglycerinester, ein oder mehrere organische Lösungsmittel und Wasser enthalten. Die Zusammensetzungen sind hervorragend zur Verdickung von tensidhaltigen Systemen geeignet.

EP 1 518 900 A2

**Beschreibung**

Verbraucherwünsche und Rheologie kosmetischer Produkte sind eng miteinander verknüpft. So wird z.B. das visuelle Erscheinungsbild einer Creme oder Lotion durch die Viskosität beeinflusst. Die sensorischen Eigenschaften, wie Konsistenz oder Verteilbarkeit bestimmen das individuelle Profil eines Kosmetikproduktes. Auch die Effektivität von Wirksubstanzen (z.B. Sonnenschutzfilter) und die Lagerstabilität der Formulierung stehen in enger Abhängigkeit zu den rheologischen Eigenschaften des Produktes. Im kosmetischen Bereich kommt daher den Verdickungsmitteln und Gelbildnern eine tragende Rolle zu.

Eine Reihe von Patentschriften beschreiben den Einsatz von Polyetherestern als Verdickungsmittel. Stand der Technik ist die Verwendung langkettiger Polyether mit Fettsäureester-Resten, beispielsweise von Polyethylenglykol 6000 Distearat (PEG 6000 Distearat). In US 4,774,017 werden Polyethylen-glycol-polypropylen-glycol Monoether als konsistenzgebende Komponente, in DE 37 26 015 die Umsetzungsprodukte von Polyalkoholen mit Fettsäuren, beispielsweise Pentaerythritolfettsäureester, und deren verdickende Wirkung, beschrieben.
US 5,129,462 beschreibt Shampoo-Formulierungen, enthaltend Polyethylenglycol-Polyolfettsäureester, insbesondere PEG Pentaerythritolfettsäureester als Verdickungsmittel.

Die Verarbeitung und Formulierbarkeit dieser Verbindungsklasse ist durch deren hohe Schmelzpunkte bzw. Stockpunkte beeinträchtigt. Bei der Verwendung fester Verdickungsmittel bei der Herstellung von tensidhaltigen kosmetischen Produkten, wie z.B. einem Shampoo oder Duschbad, muss in der Wärme gearbeitet werden. Dies bedeutet, dass der gesamte Tensid/Wasser/Verdicker-Ansatz erwärmt werden muss, um das Lösen bzw. das Schmelzen des festen Verdickungsmittels zu bewirken. Anschließend muss der Ansatz wieder auf Raumtemperatur abgekühlt werden. Dieser Vorgang ist aus zeitlichen und ökonomischen Gründen unvorteilhaft.

Es bestand daher die Aufgabe, Verdicker bereitzustellen, die in flüssiger Form vorliegen, und damit in einfacher Weise in Formulierungen eingearbeitet werden können. Zudem ist ein hoher Aktivgehalt (Feststoffgehalt) vorteilhaft. Des weiteren sollten diese flüssigen Verdickerkonzentrate bei Raumtemperatur ein homogenes, möglichst klares Aussehen aufweisen, und auch bei längerer Lagerung bei tiefen oder hohen Temperaturen keinerlei Sedimentationen oder Inhomogenitäten aufweisen.

Überraschenderweise wurde gefunden, dass die wachsartigen oxalkylierten Polyglycerinester der Formel (1)

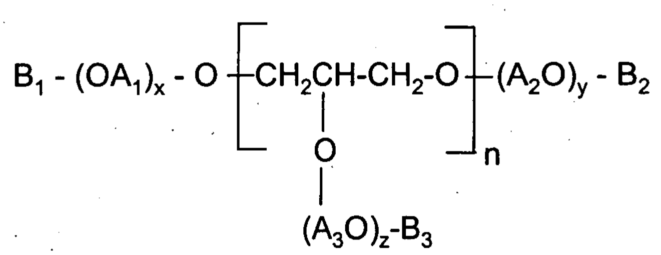

(1)

worin

| | |
|---|---|
| $A_1$, $A_2$ und $A_3$ | jeweils unabhängig voneinander eine Gruppe der Formel $-C_2H_4-$ oder $-C_3H_6-$ bedeuten, |
| $B_1$, $B_2$ oder $B_3$ | jeweils unabhängig voneinander Wasserstoff oder eine Gruppe der Formel -COR bedeuten, wobei mindestens einer der Reste $B_1$, $B_2$ oder $B_3$ eine Gruppe der Formel -COR ist, |
| R | $C_7$-$C_{21}$-Alkyl, $C_7$-$C_{21}$-Hydroxyalkyl oder $C_2$-$C_{21}$-Alkenyl ist, |
| n | im Mittel eine Zahl von 1,5 bis 10 ist, und |
| x,y und z | Zahlen von 0 bis 100 bedeuten, wobei die Summe von x, y und z 50 bis 250 beträgt, |

in hochkonzentrierter, flüssiger Form mit klarem Aussehen bereitgestellt werden können, wenn sie in einem Gemisch aus Wasser und organischem Lösungsmittel oder Wasser und organischem Lösungsmittelgemisch gelöst werden.

Gegenstand der Erfindung sind fließfähige Zusammensetzungen bzw. Konzentrate, insbesondere Verdickerkonzentrate, enthaltend

a) einen oder mehrere oxalkylierte Polyglycerinester der Formel (1)

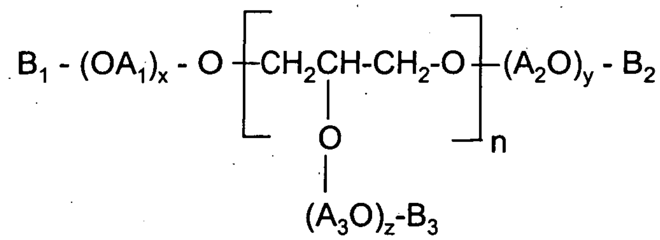

(1)

worin

A$_1$, A$_2$ und A$_3$ jeweils unabhängig voneinander eine Gruppe der Formel $-C_2H_4-$ oder $-C_3H_6-$ bedeuten,

B$_1$, B$_2$ und B$_3$ jeweils unabhängig voneinander Wasserstoff oder eine Gruppe der Formel -COR bedeuten, wobei mindestens einer der Reste $B_1$, $B_2$ oder $B_3$ eine Gruppe der Formel -COR ist,

R $C_7$-$C_{21}$-Alkyl, $C_7$-$C_{21}$-Hydroxyalkyl oder $C_2$-$C_{21}$-Alkenyl ist,

n im Mittel eine Zahl von 1,5 bis 10, vorzugsweise 1,8 bis 5, ist, und

x, y und z Zahlen von 0 bis 100 bedeuten, wobei die Summe von x, y und z 50 bis 250, vorzugsweise 100 bis 200, insbesondere 130 bis 170, beträgt,

b) ein organisches Lösungsmittel oder organisches Lösungsmittelgemisch, und

c) Wasser.

Die für den Kondensationsgrad n angegebenen Zahlen stellen zahlenmittlere Werte dar.

Die erfindungsgemäßen Zusammensetzungen enthalten ein organisches Lösungsmittel oder ein organisches Lösungsmittelgemisch.

Prinzipiell kommen als organische Lösungsmittel alle ein- oder mehrwertigen Alkohole oder Derivate von ein- oder mehrwertigen Alkoholen in Betracht, vorausgesetzt, dass sie von den Verbindungen der Formel (1) verschieden sind. Unter den Derivaten der ein- und mehrwertigen Alkohole sind Ester und Ether bevorzugt.

In einer bevorzugten Ausführungsform enthalten die erfindungsgemäßen Zusammensetzungen mindestens einen einwertigen Alkohol als organisches Lösungsmittel.

Bevorzugte einwertige Alkohole, die in den erfindungsgemäßen Zusammensetzungen als Lösungsmittel verwendet werden können, sind ausgewählt aus der Gruppe bestehend aus Methanol, Ethanol, Propanol, Isopropanol, n-Butanol, i-Butanol, t-Butanol, Hexylalkohol, Trimethylhexanol, Butyloctanol, Fettalkoholen, vorzugsweise Myristyl-, Cetyl-, Oleyl- und Stearylalkohol, Benzylalkohol, Phenylpropanol und Diacetonalkohol.

In einer weiteren bevorzugten Ausführungsform enthalten die erfindungsgemäßen Zusammensetzungen mindestens einen mehrwertigen Alkohol oder ein Derivat eines mehrwertigen Alkohols als organisches Lösungsmittel.

Bevorzugte mehrwertige Alkohole und Derivate von mehrwertigen Alkoholen, die in den erfindungsgemäßen Zusammensetzungen als Lösungsmittel verwendet werden können, sind ausgewählt aus der Gruppe bestehend aus Methoxyethanol, Ethoxyethanol, Butoxyethanol, Isobutoxypropanol, Methoxyisopropanol, Butoxyisopropanol, Phenoxyisopropanol, Methoxybutanol, vorzugsweise 4-Methoxybutanol, Methoxymethylbutanol, Glykol, Benzolglykol, Propylenglykol, Butylenglykol, Butandiol, Methylpropandiol, Pentylenglykol, Isopentyldiol, Neopentylglykol, Hexylenglykol, Hexandiol, Ethylhexandiol, Diethylenglykol, Methoxydiglykol, Ethoxydiglykol, Butoxydiglykol, Dimethoxydiglykol, Dipropylenglykol, Glycerin und 1,2,6-Hexantriol.

In einer weiteren bevorzugten Ausführungsform enthalten die erfindungsgemäßen Zusammensetzungen mindestens ein Derivat eines ein- oder mehrwertigen Alkohols ausgewählt aus ethoxylierten und/oder propoxylierten Alkoholen als Lösungsmittel. Unter diesen Derivaten bevorzugt sind ethoxylierte und/oder propoxylierte Alkohole mit 1 bis 40 Mol, vorzugsweise 1 bis 30 Mol, besonders bevorzugt 1 bis 20 Mol Ethylenoxid und/oder Propylenoxid pro 1 Mol des zugrundeliegenden ein- oder mehrwertigen Alkohols. Insbesondere bevorzugt sind Polypropylenglycol-7 und Polypropylenglycol-10.

In einer weiteren bevorzugten Ausführungsform enthalten die erfindungsgemäßen Zusammensetzungen mindestens ein organisches Lösungsmittel auf Basis von pflanzlichen Ölen bzw. auf Basis von Mono-, Di- oder Triglyceriden, also Verbindungen auf Basis von Glycerin und Fettsäuren, oder auf Basis von Mischungen dieser Glyceride. Insbesondere ethoxylierte und/oder propoxylierte, vorzugsweise ethoxylierte, Derivate von Mono-, Di- oder Triglyceriden, die auch solche Verbindungen umfassen, in denen sich Ethylenoxid- und/oder Propylenoxid-Einheiten zwischen

den Glycerin- und den Säureeinheiten befinden können, oder Mischungen dieser Glyceride, die zudem bei Raumtemperatur flüssig sind, eignen sich hervorragend als Lösungsmittel. In einer besonders bevorzugten Ausführungsform enthalten die ethoxylierten und/oder propoxylierten Derivate der Mono-, Di- und Triglyceride jedoch keine Ethylenoxid- und/oder Propylenoxid-Einheiten zwischen den Glycerin- und den Säureeinheiten.

In einer weiteren bevorzugten Ausführungsform enthalten die erfindungsgemäßen Zusammensetzungen als Lösungsmittel mindestens ein Derivat des Monoglycerins, worin an eine, zwei oder drei der Hydroxylgruppen des Monoglycerins über eine Etherbrücke ein Polyalkylenglykolrest gebunden ist, der aus Ethylenoxid- und/oder Propylenoxideinheiten, vorzugsweise Ethylenoxideinheiten aufgebaut ist, und wobei diese Derivate zwischen 1 und 40 Mol, vorzugsweise zwischen 1 und 30 Mol und besonders bevorzugt zwischen 1 und 20 Mol Alkylenoxid pro 1 Mol Monoglycerin enthalten und wobei eine, zwei oder drei der Hydroxylgruppen dieser Verbindungen verestert und die Esterreste von gesättigten, ungesättigten, geradkettigen oder verzweigten Carbonsäuren mit 6 bis 22 C-Atomen abgeleitet sind. Weiterhin bevorzugt besteht das Lösungsmittel aus einer Mischung von den soeben genannten Derivaten des Monoglycerins.

In einer weiteren bevorzugten Ausführungsform enthalten die erfindungsgemäßen Zusammensetzungen als Lösungsmittel mindestens ein Derivat des Monoglycerins, worin eine, zwei oder drei der Hydroxylgruppen des Monoglycerins verestert und die Esterreste von gesättigten, ungesättigten, geradkettigen oder verzweigten Carbonsäuren mit 6 bis 22 C-Atomen abgeleitet sind, worin an eine oder zwei der nicht veresterten Hydroxylgruppen des Monoglycerins über eine Etherbrücke ein Polyalkylenglykolrest gebunden ist, der aus Ethylenoxid- und/oder Propylenoxideinheiten, vorzugsweise Ethylenoxideinheiten aufgebaut ist, und wobei diese Derivate zwischen 1 und 40 Mol, vorzugsweise zwischen 1 und 30 Mol und besonders bevorzugt zwischen 1 und 20 Mol Alkylenoxid pro 1 Mol Monoglycerin enthalten. Weiterhin bevorzugt besteht das Lösungsmittel aus einer Mischung von den soeben genannten Derivaten des Monoglycerins.

In einer weiteren bevorzugten Ausführungsform enthalten die erfindungsgemäßen Zusammensetzungen als Lösungsmittel mindestens eine Verbindung der Formel (1), worin jedoch n 1 ist und die Summe von x, y und z 1 bis 40, vorzugsweise 1 bis 30 und besonders bevorzugt 1 bis 20 beträgt. Diese Verbindungen werden im folgenden als Verbindungen der Formel (2) bezeichnet.

Unter den Derivaten des Glycerins als Lösungsmittel besonders bevorzugt sind PEG-6 Caprylic/Capric Glycerides (z.B. Softigen 767, Sasol), d.h. Polyethylenglycol-Derivate einer Mischung aus Mono-, Di- und Triglyceriden aus Capryl- und Caprinsäuren mit im Durchschnitt 6 Mol Ethylenoxid und PEG-7 Glyceryl Cocoate (z.B. Cetiol HE, Cognis), d.h. ein Polyethylenglycolether von Glycerylcocoat mit im Durchschnitt 7 Mol Ethylenoxid pro 1 Mol Glycerin.

Die Herstellung der oxalkylierten Polyglycerinester gemäß Formel (1) erfolgt entweder durch Oxalkylierung eines Polyglycerins und anschließende Veresterung oder durch Veresterung des Polyglycerins und anschließende Oxalkylierung.

Die erfindungsgemäß eingesetzten alkoxylierten Polyglycerinester werden in mehreren Reaktionsstufen hergestellt. Die Synthese der Polyglycerine bzw. Oligoglycerine, Diglycerin, Triglycerin, Tertraglycerin bis Decaglycerin erfolgt in bekannter Weise durch Polykondensation von Glycerin in Gegenwart von Katalysatoren, beispielsweise reduzierende Phosphorsäuren, Alkalihydroxide, Alkalicarbonate, Alkalibicarbonate, Alkalialkoholate und Alkoxide bei Temperaturen von 190 bis 270°C. Unter Austrag von Kondensationswasser erfolgt innerhalb von 8 bis 72 Stunden die Bildung der Polyglycerine. Die Hydroxylzahl (OH-Zahl) eines solchen Reaktionsgemisches liegt beispielsweise bei 1072 mg KOH/g für ein Oligomerengemisch, das im Mittel einem Polyglycerin-4 entspricht. Die Zahl n als Maß für den Kondensationsgrad liegt zwischen 1,5 bis 10, insbesondere zwischen 1,8 und 5.

Die Alkoxylierung, insbesondere die Ethoxylierung der Polyglycerine bzw. Oligoglycerine, erfolgt bei 130 bis 190°C, bevorzugt bei 160°C, in Gegenwart eines basischen Katalysators, beispielsweise NaOH, nach Trocknung bei 100°C und 20 mbar Vakuum (ca. 0,5 Stunden), wobei Alkoxid, vorzugsweise Ethylenoxid bei einem Druck von 1 bis 6 bar, insbesondere bei 4 bis 6 bar, innerhalb von 15 Stunden zudosiert wird. Die erhaltenen ethoxylierten Polyglycerine bzw. Oligoglycerine haben einen Gesamt-EO-Grad (x+y+z) von 50 bis 250, bevorzugt 100 bis 200, besonders bevorzugt 130 bis 170 bezogen auf die mittlere Molekularmasse des Polyglycerins.

Das hergestellte ethoxylierte Poly-/Oligoglycerin wird nach Abkühlen des Reaktionsgemisches auf 60 bis 100°C mit einem Katalysator, z.B. Alkylbenzolsulfonsäure, versetzt und der pH-Wert durch Zusatz von Säure, bevorzugt unterphosphoriger Säure oder Phosphorsäure, auf 4 bis 5 (10 % wässrig) eingestellt. Anschließend wird durch Zusatz einer Fettsäure, beispielsweise Stearinsäure, Isostearinsäure, 12-Hydroxystearinsäure, Kokosfettsäure, Laurinsäure, Ölsäure oder deren Alkylester, Chloriden oder Anhydriden bei einer Reaktionstemperatur von 160 bis 230°C und einer Reaktionsdauer von 10 bis 35 Stunden verestert. Der molare Anteil an Fettsäure bzw. Fettsäurederivaten kann beliebig gewählt werden, mindestens eine OH-Gruppe des oxalkylierten Polyglycerins muss aber verestert sein.

Die Herstellweise dieser alkoxylierten Poly-/Oligoglycerin-Fettsäureester kann auch so variiert werden, dass die Poly-/Oligoglycerine zuerst im entsprechenden Molverhältnis mit Fettsäure verestert und dann alkoxyliert werden.

Bedingt durch dieses Herstellverfahren handelt es sich bei den Polyglycerinderivaten um Mischungen von Verbindungen der oben genannten Formel mit unterschiedlichem Wert für n, d.h. es kommen auch Gemische mit einem

Gehalt an Monoglycerinester in Frage.

Die oben genannten alkoxylierten Polyglycerinester sind bei Raumtemperatur überwiegend Wachse, die mit einem Gemisch aus Wasser und organischem Lösungsmittel bzw. Wasser und organischem Lösungsmittelgemisch die erfindungsgemäßen fließbaren, gut handhabbaren Produkte mit einem klaren Aussehen ergeben. Die Zugabe der (gegebenenfalls erwärmten) Lösungsmittel erfolgt vorteilhafterweise direkt zu den aufgeschmolzenen alkoxylierten Polyglycerinestern, bevorzugt bei 60 bis 100°C, besonders bevorzugt bei 80°C.

Weiterer Gegenstand der vorliegenden Erfindung ist somit auch die Verwendung eines oxalkylierten Polyglycerinesters der Formel (1), mindestens eines organischen Lösungsmittels oder eines organischen Lösungsmittelgemischs und Wasser zur Herstellung der erfindungsgemäßen Zusammensetzungen.

Versuche, die wachsartigen alkoxylierten Polyglycerinester der Formel (1) in Wasser oder in wasserfreien organischen Lösungsmitteln zu lösen, führten zu unbefriedigenden Ergebnissen. Die Lösungen waren inhomogen mit trübem Aussehen (s. Tabelle 1 ).

Tabelle 1

| (Angaben in Gew.-%) | | | | | |
|---|---|---|---|---|---|
| | A | B | C | D | E |
| Verdicker | 60 | 60 | 60 | 60 | 60 |
| E-Wasser | 40 | 20 | - | - | 30 |
| Cetiol HE | - | 20 | 40 | - | - |
| Softigen 767 | - | - | - | 40 | 10 |
| Aussehen | 1 | 2 | 3 | 3 | 2 |

1: trüb, hochviskos;
2: klar, niedrig viskos;
3: Ausflockung, fest

Als Verdicker in den in Tabelle 1 wiedergegebenen Versuchen wurde Diglycerin-148 EO-Tristearat verwendet, d.h. eine Verbindung gemäß Formel (1), in der n = 2, A = $C_2H_4$, x + y + z = ca. 148 und B = Stearyl bedeutet.

Versuchsbeschreibung:

Das Diglycerin-148 EO-Tristearat wird auf 80°C erwärmt. Danach wird das Lösungsmittel in einer Menge entsprechend den in Tabelle 1 genannten Gewichtsprozenten zugegeben. Anschließend wird die erhaltene Lösung auf Raumtemperatur abgekühlt.

Chemische Bezeichnung der eingesetzten Handelsprodukte:

Cetiol HE: PEG-7 Glyceryl Cocoat
Softigen 767: PEG-6 Caprylic/Capric Glycerid

Die erfindungsgemäßen Zusammensetzungen enthalten, bezogen auf die gesamte Zusammensetzung, 10 bis 95 Gew.-%, bevorzugt 20 bis 80 Gew.-% und besonders bevorzugt 30 bis 70 Gew.-% alkoxylierte Polyglycerinsäureester der Formel (1).

In den erfindungsgemäßen Zusammensetzungen beträgt das Gewichtsverhältnis von Wasser zu organischem Lösungsmittel oder organischem Lösungsmittelgemisch vorzugsweise von 3 zu 1 bis 1 zu 3.

Die erfindungsgemäßen Zusammensetzungen eignen sich als Verdicker und Dispergiermittel für wässrige, wässrig-alkoholische und wässrig-tensidische Zubereitungen und als Emulgatoren oder Suspendiermittel mit verdikkender Wirkung und Konsistenzgeber für Emulsionen und Suspensionen. Insbesondere eignen sie sich als Verdicker in tensidhaltigen Systemen.

Weiterer Gegenstand der Erfindung ist daher auch die Verwendung einer erfindungsgemäßen Zusammensetzung als Verdicker in tensidhaltigen Systemen.

Bei den Zubereitungen, Emulsionen und Suspensionen enthaltend eine erfindungsgemäße Zusammensetzung handelt es sich um kosmetische, dermatologische und pharmazeutische Mittel.

Weiterer Gegenstand der vorliegenden Erfindung ist daher auch die Verwendung einer erfindungsgemäßen Zusammensetzung zur Herstellung eines kosmetischen, dermatologischen oder pharmazeutischen Mittels sowie ein kosmetisches, dermatologisches oder pharmazeutisches Mittel enthaltend eine erfindungsgemäße Zusammensetzung.

Vorzugsweise liegen die kosmetischen, dermatologischen oder pharmazeutischen Mittel als Shampoos, Duschbäder, Duschgels, Schaumbäder, Gels, Lotions, Cremes oder Salben vor.

Die erfindungsgemäßen kosmetischen, dermatologischen oder pharmazeutischen Mittel enthalten, bezogen auf die fertige Formulierung, bevorzugt 0,05 bis 10 Gew.-%, besonders bevorzugt 0,1 bis 5 Gew.-%, insbesondere bevorzugt 0,5 bis 3 Gew.-%, der Polyglycerinester der Formel (1).

Die erfindungsgemäßen kosmetischen, dermatologischen oder pharmazeutischen Mittel können als weitere Hilfs- und Zusatzstoffe alle üblichen anionischen, kationischen, zwitterionischen, nicht-ionischen und amphoteren Tenside, sowie weitere in der Kosmetik gebräuchliche Zusätze, wie z.B. Überfettungsmittel, Stabilisatoren, biogene Wirkstoffe, Glycerin, Konservierungsmittel, Perlglanzmittel, Farb- und Duftstoffe, Lösungsmittel, Trübungsmittel, weitere Verdickungsmittel und Dispergiermittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen kosmetischen, dermatologischen oder pharmazeutischen Mitteln antimikrobiell wirkende Agentien zugesetzt werden

Die Gesamtmenge der in den erfindungsgemäßen kosmetischen, dermatologischen oder pharmazeutischen Mitteln eingesetzten Tenside kann, bezogen auf das fertige Mittel, zwischen 5 bis 70 Gew.-%, bevorzugt zwischen 10 und 40 Gew.-%, besonders bevorzugt zwischen 12 und 35 Gew.-%, bezogen auf 100 % Wirksubstanz, betragen.

Als anionische waschaktive Substanzen seien genannt: ($C_{10}$-$C_{20}$)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, $\alpha$-Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Der Gewichtsanteil der anionischen Tenside in den erfindungsgemäßen kosmetischen, dermatologischen oder pharmazeutischen Mitteln liegt bevorzugt im Bereich von 7 bis 30 Gew.-%, besonders bevorzugt 10 bis 25 Gew.-%, insbesondere bevorzugt 12 bis 22 Gew.-%.

Geeignete kationische Tenside sind beispielsweise quartäre Ammonium-Salze wie Di-($C_{10}$-$C_{24}$)-Alkyl-dimethylammoniumchlorid oder -bromid, vorzugsweise Di-($C_{12}$-$C_{18}$)-Alkyl-dimethylammoniumchlorid oder -bromid; ($C_{10}$-$C_{24}$)-Alkyl-dimethylethylammoniumchlorid oder -bromid; ($C_{10}$-$C_{24}$)-Alkyl-trimethylammoniumchlorid oder -bromid, vorzugsweise Cetyltrimethylammoniumchlorid oder -bromid und ($C_{20}$-$C_{22}$)-Alkyl-trimethylammoniumchlorid oder -bromid; ($C_{10}$-$C_{24}$)-Alkyldimethylbenzyl-ammoniumchlorid oder -bromid, vorzugsweise ($C_{12}$-$C_{18}$)-Alkyldimethylbenzylammoniumchlorid; N-($C_{10}$-$C_{18}$)-Alkyl-pyridiniumchlorid oder -bromid, vorzugsweise N-($C_{12}$-$C_{16}$)-Alkyl-pyridiniumchlorid oder -bromid; N-($C_{10}$-$C_{18}$)-Alkylisochinolinium-chlorid, -bromid oder -monoalkylsulfat; N-($C_{12}$-$C_{18}$)-Alkylpolyoylaminoformylmethyl-pyridiniumchlorid; N-($C_{12}$-$C_{18}$)-Alkyl-N-methylmorpholinium-chlorid, -bromid oder -monoalkylsulfat; N-($C_{12}$-$C_{18}$)-Alkyl-N-ethylmorpholinium-chlorid, -bromid oder -monoalkylsulfat; ($C_{16}$-$C_{18}$)-Alkyl-pentaoxethylammonium-chlorid; Diisobutyl-phenoxyethoxyethyldimethylbenzylammonium-chlorid; Salze des N,N-Diethylaminoethylstearylamids und -oleylamids mit Salzsäure, Essigsäure, Milchsäure, Zitronensäure, Phosphorsäure; N-Acyl-aminoethyl-N, N-diethyl-N-methyl-ammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside in den erfindungsgemäßen kosmetischen, dermatologischen oder pharmazeutischen Mitteln liegt bevorzugt im Bereich von 1 bis 10 Gew.-%, besonders bevorzugt 2 bis 7 Gew.-%, insbesondere bevorzugt 3 bis 5 Gew.-%.

Als nichtionische Tenside, kommen beispielsweise in Betracht: Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics® ); Fettsäurealkylolamide, (Fettsäureamidpolyethylenglykole); N-Alkyl-, N-Alkoxypolyhydroxyfettsäureamid, Saccharoseester; Sorbitester und der Polyglykolether.

Der Gewichtsanteil der nichtionischen Tenside in den erfindungsgemäßen kosmetischen, dermatologischen oder pharmazeutischen Mitteln liegt bevorzugt im Bereich von 1 bis 20 Gew.-%, besonders bevorzugt 2 bis 10 %, insbesondere bevorzugt 3 bis 7 Gew.-%.

Bevorzugte Amphotenside sind: N-($C_{12}$-$C_{18}$)-Alkyl-$\beta$-aminopropionate und N-($C_{12}$-$C_{18}$)-Alkyl-$\beta$-iminodipro-

pionate als Alkali- und Mono-, Di- und Trialkylammonium-Salze; N-Acylaminoalkyl-N,N-dimethyl-acetobetain, vorzugsweise N-($C_8$-$C_{18}$)-Acyl-aminopropyl-N,N-dimethylacetobetain; ($C_{12}$-$C_{18}$)-Alkyl-dimethylsulfopropyl-betain; Amphotenside auf Basis Imidazolin (Handelsname: Miranol® , Steinapon® ), vorzugsweise das Natrium-Salz des 1-(β-Carboxymethyloxyethyl)-1-(carboxymethyl)-2-lauryl-imidazoliniums; Aminoxid, z.B. ($C_{12}$-$C_{18}$)-Alkyldimethylaminoxid, Fettsäureamidoalkyldimethylaminoxid.

Der Gewichtsanteil der amphoteren Tenside in den erfindungsgemäßen kosmetischen, dermatologischen oder pharmazeutischen Mitteln liegt bevorzugt im Bereich von 0,5 bis 20 Gew.-%, besonders bevorzugt 1 bis 10 Gew.-%.

Des weiteren können in den erfindungsgemäßen kosmetischen, dermatologischen oder pharmazeutischen Mitteln schaumverstärkende Co-Tenside aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Bevorzugte Tenside in den erfindungsgemäßen kosmetischen, dermatologischen oder pharmazeutischen Mitteln sind Laurylsulfat, Laurethsulfat, Cocoamidipropylbetain, Natriumcocoylglutamat, Di-natriumlaurethsulfosuccinat und Cocosfettsäurediethanolamid.

Die erfindungsgemäßen kosmetischen, dermatologischen oder pharmazeutischen Mittel können außerdem weitere in der Kosmetik gebräuchliche Zusätze wie Überfettungsmittel, Stabilisatoren, biogene Wirkstoffe, Glycerin, Konservierungsmittel, Perlglanzmittel, Farb- und Duftstoffe, Lösungsmittel, Trübungsmittel, Verdickungsmittel und Dispergiermittel ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen enthalten. Des weiteren können den erfindungsgemäßen kosmetischen, dermatologischen oder pharmazeutischen Mitteln antimikrobiell wirkende Agentien zugesetzt werden.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für Fette sind Glyceride, als Wachse kommen u.a. Bienenwachs, Paraffinwachs oder Mikrowachse, gegebenenfalls in Kombination mit hydrophilen Wachsen, z.B. Cetylstearylalkohol in Frage.

Als Stabilisatoren können Metallsalze von Fettsäuren, wie z.B. Magnesium-, Aluminium- und/oder Zinkstearat eingesetzt werden.

Unter biogenen Wirkstoffen sind beispielsweise Pflanzenextrakte und Vitaminkomplexe zu verstehen.

Als Konservierungsmittel eignen sich beispielsweise Phenoxyethanol, Formaldehydlösung, Parabene, Pentandiol oder Sorbinsäure.

Als Perlglanzmittel kommen beispielsweise Glycoldistearinsäureester, wie Ethylenglycoldistearat, aber auch Fettsäuremonoglykolester in Betracht.

Als Farbstoffe können die für kosmetische Zwecke geeigneten und zugelassenen Substanzen verwendet werden.

Als weitere Verdickungsmittel eignen sich Natrium-, Kalium-, Ammoniumchlorid, Natriumsulfat, Fettsäurealkylolamide, Cellulosederivate, beispielsweise Hydroxyethylcellulose, Guar Gum, Polyvinylalkohol, Polyvinylpyrrolidon, Hydroxypropyl guar gum, Stärke und Stärkederivate sowie natürliche Gummen, Carboxyvinylpolymere, beispielsweise Carbopol 934, -940, -941, -956, -980, -981, -1342, -1382, Ethylenglykolester von Fettsäuren mit 14 bis 22, besonders bevorzugt 16 bis 22 Kohlenstoffatomen, insbesondere Mono- und Di-ethylenglycolstearat. Bevorzugt sind auch Stearinmonoethanolamid, Stearindiethanolamid, Stearinisopropanolamid, Stearinmonoethanolamidstearat, Stearylstearat, Cetylpalmitat, Glycerylstearat, Stearamiddiethanolamiddistearat, Stearamidmonoethanolamidstearat, N,N-Dihydrocarbyl ($C_{12}$-$C_{22}$)- insbesondere ($C_{16}$-$C_{18}$)-amidobenzoesäure und deren lösliche Salze, N,N-di($C_{16}$-$C_{18}$)-amidobenzoesäure und deren Derivate.

Die Dispergiermittel werden, bezogen auf das fertige kosmetische, dermatologische oder pharmazeutische Mittel, in Konzentrationen von bevorzugt 0,5 bis 10 Gew.-%, besonders bevorzugt von 0,5 bis 5 Gew.-%, insbesondere bevorzugt von 1 bis 4 Gew.-%, eingesetzt.

Die gewünschte Viskosität der kosmetischen, dermatologischen oder pharmazeutischen Mittel kann durch Zugabe von Wasser und/oder organischen Lösungsmitteln oder durch Zugabe einer Kombination aus organischen Lösungsmitteln und Verdickungsmitteln eingestellt werden.

Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, 1-Butanol und t-Butanol eingesetzt.

Weiterhin bevorzugt sind einwertige Alkohole, besonders bevorzugt solche ausgewählt aus Methanol, Ethanol, Propanol, Isopropanol, n-Butanol, i-Butanol, t-Butanol, Hexylalkohol, Trimethylhexanol, Butyloctanol, Oleylalkohol, Benzylalkohol, Phenylpropanol und Diacetonalkohol, insbesondere bevorzugt solche mit 1 bis 6 C-Atomen, außerordentlich bevorzugt Methanol, Ethanol, Propanol, Isopropanol, n-Butanol, 1-Butanol und t-Butanol.

Bevorzugt sind ferner mehrwertige Alkohole und Derivate von mehrwertigen Alkoholen, besonders bevorzugt solche ausgewählt aus der Gruppe Methoxyethanol, Ethoxyethanol, Butoxyethanol, Isobutoxypropanol, Methoxyisopropanol, Butoxyisopropanol, Phenoxyisopropanol, Methoxybutanol, vorzugsweise 4-Methoxybutanol, Methoxymethylbutanol, Glykol, Benzolglykol, Propylenglykol, Butylenglykol, Methylpropandiol, Pentylenglykol, Isopentyldiol, Neopentylglykol, Hexylenglykol, Hexandiol, Ethylhexandiol, Diethylenglykol, Methoxydiglykol, Ethoxydiglykol, Butoxydiglykol, Dimethoxydiglykol, Dipropylenglykol, Glycerin und 1,2,6-Hexantriol, ganz besonders bevorzugt Glykol, Propylenglykol, Butylenglykol und Glycerin.

Bevorzugt sind ferner Ketone, Ester, Ether, Amide, Sulfoxide, Nitrile, O-, N- und S-Heterocyclen, bevorzugt Aceton, Methoxyethanolacetat, Triacetin (Glycerintriacetat), Amylacetat, Benzylbenzoat, Benzyllaurat, Butoxyethylacetat, Butylacetat, Butylenglykolpropionat, Butyllactat, Butyloctylbenzoat, Butyloctylsalicylat, Butyrolacton, $C_{5}$-$_{18}$-Fettsäuretriglyceride, PEG/PPG-Copolymere (PEG: Polyethylenglykol; PPG: Polypropylenglykol), Propylacetat, Propylencarbonat, Propylenglykolbutylether, Propylenglycolpropylether, Tetrahydrofurfurylacetat, Tetrahydrofurfurylalkohol, Thiolandiol, Tributylcitrat, Tributylcresylbutan, Acetonitril, THF (Tetrahydrofuran), DMF (Dimethylformamid), DMSO (Dimethylsulfoxid), DBU (Diazabicycloundecan), Pyridin, besonders bevorzugt Aceton, Acetonitril, THF (Tetrahydrofuran) und DMF (Dimethylformamid).

Bevorzugt sind ferner ethoxylierte und/oder propoxylierte Alkohole, besonders bevorzugt ethoxylierte und/ oder propoxylierte Alkohole mit 1 bis 40, vorzugsweise 1 bis 30, besonders bevorzugt 1 bis 20 Mol Ethylenoxid und/ oder Propylenoxid, insbesondere bevorzugt ethoxylierte und/oder propoxylierte Alkohole ausgewählt aus Polypropylenglycol-7 (PPG-7: im Mittel enthaltend 7 Propylenglykol-Einheiten), Polypropylenglycol-10, PPG-2-Buteth-3, PPG-3-Buteth-5, PPG-5-Buteth-7, PPG-7-Buteth-10, PPG-12-Buteth-16, PPG-15-Buteth-20, PPG-20-Buteth-20, PPG-2-Butylether, PPG-3-Butylether, PPG-24-Glycereth-24, PPG-10-Glycerylether, Glycerylether, PPG-2-Methylether, PPG-3-Methylether, PPG-2-Methyletheracetat, PPG-2-Propylether, Propylenglykolbutylether, Propylenglykolpropylether, Methoxy PEG-10, Methoxy PEG-16, Buteth-3, Sorbeth-6 und Sorbeth-20.

Als Trägermaterialien in Betracht kommen pflanzliche Öle, natürliche und gehärtete Öle, Wachse, Fette, Wasser, Alkohole, Polyole, Glycerol, Glyceride, flüssige Paraffine, flüssige Fettalkohole, Sterol, Polyethylenglykole, Cellulose und Cellulose-Derivate.

Im Fall, dass die erfindungsgemäßen kosmetischen, dermatologischen und pharmazeutischen Mittel Emulsionen sind, liegt der nichtwässrige Anteil der Emulsionen, der sich weitgehend aus dem Emulgator, dem Verdicker und dem Ölkörper zusammensetzt, üblicherweise bei 5 bis 95 %, vorzugsweise bei 15 bis 75 Gew.-%. Daraus folgt, dass die Emulsionen 5 bis 95 Gew.-% und vorzugsweise 25 bis 85 Gew.-% Wasser enthalten können, abhängig davon, ob Lotionen mit einer vergleichsweise niedrigen oder Cremes und Salben mit höher Viskosität hergestellt werden sollen.

Die Emulsionen können als Hautpflegemittel, wie beispielsweise Tagescremes, Nachtcremes, Pflegecremes, Nährcreme, Bodylotions, Salben und dergleichen eingesetzt werden und als weitere Hilfs- und Zusatzstoffe, Ölkörper, Co-Emulgatoren, Überfettungsmittel, Fette, Wachse, Stabilisatoren, biogene Wirkstoffe, Glycerin, Konservierungsmittel, Farb- und Duftstoffe enthalten.

Als Ölkörper kommen beispielsweise Guerbetalkohole auf Basis von Fettalkoholen mit 6 bis 18, vorzugsweise 8 bis 10 Kohlenstoffatomen, Ester von linearen ($C_6$-$C_{13}$)-Fettsäuren mit linearen ($C_6$-$C_{20}$) Fettalkoholen, Ester von verzweigten ($C_6$-$C_{13}$)-Carbonsäuren mit linearen ($C_6$-$C_{20}$)-Fettalkoholen, Ester von linearen ($C_6$-$C_{18}$)-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis ($C_6$-$C_{10}$) -Fettsäuren, pflanzliche Öle, verzweigte primäre Alkohole, substituierte Cyclohexane, Guerbetcarbonate, Dialkylether und/oder aliphatische bzw. aromatische Kohlenwasserstoffe in Betracht. Der Anteil der Ölkörper am nichtwässrigen Anteil der Emulsionen kann 5 bis 95 und vorzugsweise 15 bis 75 Gew.-% ausmachen.

Als nichtionogene Co-Emulgatoren kommen u.a. in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; ($C_{12}$-$C_{18}$) -Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen.

Als ionogene Co-Emulgatoren eignen sich z.B. anionische Emulgatoren, wie mono-, di- oder tri-Phosphorsäureester, aber auch kationische Emulgatoren wie mono-, di- und tri-Alkylquats und deren polymere Derivate.

Um die rheologischen Eigenschaften von wässrigen oder lösungsmittelhaltigen Emulsionen oder Suspensionen einzustellen, werden in der Fachliteratur eine Vielzahl von unterschiedlichen Systemen angegeben. Bekannt sind beispielsweise Celluloseether und andere Cellulosederivate (z.B. Carboxymethylcellulose, Hydroxyethylcellulose),

Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z.B. Polyvinylalkohole, Polyacrylamide, Polyvinylamide, Polysulfonsäuren, Polyacrylsäure, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den oben angegebenen Verbindungen, einschließlich ihrer verschiedenen Salze und Ester. Diese Polymere können wahlweise vernetzt oder unvernetzt sein.

Die Herstellung der Emulsionen kann in bekannter Weise, d.h. beispielsweise durch Heiß-, Heiß/Kalt- bzw. PIT-Emulgierung erfolgen.

Beispiel 1

Unter Verwendung des Konzentrats E aus Tabelle 1 wird folgende Waschlotion hergestellt.

| | | Gew.-% |
|---|---|---|
| A | Hostapon® KCG | 30,0 |
| B | Duftstoff | 0,3 |
| C | Wasser | ad 100 |
| | Konzentrat E aus Tabelle 1 | 1,0 |
| | Farbstoff-Lösung | 1,0 |
| | Konservierungsmittel | q.s. |
| | Genagen® CAB | 10,0 |

Herstellung

Zunächst wird Phase B in Phase A gelöst. Anschließend werden zu der Lösung nacheinander die Komponenten der Phase C zugegeben.

Die Viskosität der Waschlotion ist 6500 mPas (20°C, Brookfieldviskosimeter, 20 U/min).

Eine analoge Zusammensetzung, die sich lediglich dadurch unterscheidet, dass sie das Konzentrat E aus Tabelle 1 nicht enthält, weist eine Viskosität von lediglich 25 mPas auf.

INCl-Bezeichnung der eingesetzten Handelsprodukte:

| | |
|---|---|
| Hostapon® KCG (Clariant) | Sodium Cocoyl Glutamate |
| Genagen® CAB (Clariant) | Cocamidopropyl Betaine |

**Patentansprüche**

1. Zusammensetzung, enthaltend

    a) einen oder mehrere oxalkylierte Polyglycerinester der Formel (1)

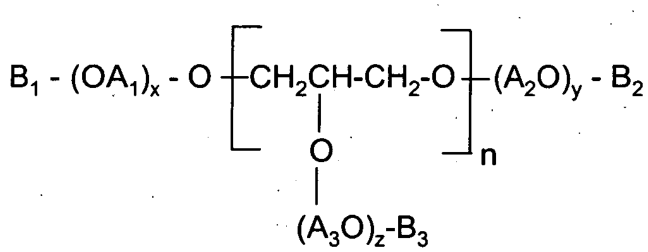

(1)

    worin

| | |
|---|---|
| $A_1$, $A_2$ und $A_3$ | jeweils unabhängig voneinander eine Gruppe der Formel $-C_2H_4-$ oder $-C_3H_6-$ bedeuten, |
| $B_1$, $B_2$ und $B_3$ | jeweils unabhängig voneinander Wasserstoff oder eine Gruppe der Formel -COR bedeuten, wobei mindestens einer der Reste $B_1$, $B_2$ oder $B_3$ eine Gruppe der Formel -COR ist, |
| R | $C_7$-$C_{21}$-Alkyl, $C_7$-$C_{21}$-Hydroxyalkyl oder $C_2$-$C_{21}$-Alkenyl ist, |
| n | im Mittel eine Zahl von 1,5 bis 10 ist, und |
| x, y und z | Zahlen von 0 bis 100 bedeuten, wobei die Summe von x, y und z 50 bis 250 beträgt. |

b) ein organisches Lösungsmittel oder organisches Lösungsmittelgemisch, und

c) Wasser.

**2.** Zusammensetzung nach Anspruch 1, **dadurch gekennzeichnet, dass** sie einen oder mehrere oxalkylierte Polyglycerinester der Formel (1) enthält, worin die Summe von x, y und z 100 bis 200 beträgt.

**3.** Zusammensetzung nach Anspruch 2, **dadurch gekennzeichnet, dass** sie einen oder mehrere oxalkylierte Polyglycerinester der Formel (1) enthält, worin die Summe von x, y und z 130 bis 170 beträgt.

**4.** Zusammensetzung nach einem oder mehreren der Ansprüche 1 bis 3, **dadurch gekennzeichnet, dass** sie oxalkylierte Polyglycerinester der Formel (1) enthält, worin n im Mittel eine Zahl von 1,8 bis 5 ist.

**5.** Zusammensetzung nach einem oder mehreren der Ansprüche 1 bis 4, **dadurch gekennzeichnet, dass** sie, bezogen auf die gesamte Zusammensetzung, 10 bis 95 Gew.-% Polyglycerinester der Formel (1) enthält.

**6.** Zusammensetzung nach einem oder mehreren der Ansprüche 1 bis 5, **dadurch gekennzeichnet, dass** sie mindestens einen von den Verbindungen der Formel (1) verschiedenen ein- oder mehrwertigen Alkohol oder mindestens ein von den Verbindungen der Formel (1) verschiedenes Derivat eines ein- oder mehrwertigen Alkohols als organisches Lösungsmittel enthält.

**7.** Zusammensetzung nach einem oder mehreren der Ansprüche 1 bis 5, **dadurch gekennzeichnet, dass** sie mindestens ein von den Verbindungen der Formel (1) verschiedenes Derivat eines ein- oder mehrwertigen Alkohols ausgewählt aus Ethern und Estern als organisches Lösungsmittel enthält.

**8.** Zusammensetzung nach einem oder mehreren der Ansprüche 1 bis 5, **dadurch gekennzeichnet, dass** sie mindestens ein von den Verbindungen der Formel (1) verschiedenes Derivat eines ein- oder mehrwertigen Alkohols als organisches Lösungsmittel enthält, wobei das Derivat ausgewählt ist aus ethoxylierten und/oder propoxylierten Alkoholen, die 1 bis 40 Mol Ethylenoxid und/oder Propylenoxid pro 1 Mol des zugrundeliegenden ein- oder mehrwertigen Alkohols enthalten.

**9.** Zusammensetzung nach einem oder mehreren der Ansprüche 1 bis 5, **dadurch gekennzeichnet, dass** sie mindestens ein von den Verbindungen der Formel (1 ) verschiedenes organisches Lösungsmittel auf Basis von pflanzlichen Ölen oder auf Basis von Mono-, Di- oder Triglyceriden oder auf Basis von Mischungen dieser Glyceride enthält.

**10.** Zusammensetzung nach Anspruch 9, **dadurch gekennzeichnet, dass** das Lösungsmittel ausgewählt ist aus ethoxylierten und/oder propoxylierten Derivaten von Mono-, Di- oder Triglyceriden oder Mischungen dieser Glyceride, die zudem bei Raumtemperatur flüssig sind.

**11.** Zusammensetzung nach Anspruch 10, **dadurch gekennzeichnet, dass** die ethoxylierten und/oder propoxylierten Derivate der Mono-, Di- und Triglyceride keine Ethylenoxid- und/oder Propylenoxid-Einheiten zwischen den Glycerin- und den Säureeinheiten enthalten.

**12.** Zusammensetzung nach einem oder mehreren der Ansprüche 1 bis 5, **dadurch gekennzeichnet, dass** sie mindestens ein Derivat eines mehrwertigen Alkohols als organisches Lösungsmittel enthält, wobei das Derivat ausgewählt ist aus Derivaten des Monoglycerins, worin an eine, zwei oder drei der Hydroxylgruppen des Monoglycerins über eine Etherbrücke ein Polyalkylenglykolrest gebunden ist, der aus Ethylenoxid- und/oder Propylenoxideinheiten aufgebaut ist, und wobei diese Derivate zwischen 1 und 40 Mol Alkylenoxid pro 1 Mol Monoglycerin enthalten und wobei eine, zwei oder drei der Hydroxylgruppen dieser Verbindungen verestert und die Esterreste von gesättigten, ungesättigten, geradkettigen oder verzweigten Carbonsäuren mit 6 bis 22 C-Atomen abgeleitet sind.

**13.** Zusammensetzung nach einem oder mehreren der Ansprüche 1 bis 5, **dadurch gekennzeichnet, dass** sie mindestens ein Derivat eines mehrwertigen Alkohols als organisches Lösungsmittel enthält, wobei das Derivat ausgewählt ist aus Derivaten des Monoglycerins, worin eine, zwei oder drei der Hydroxylgruppen des Monoglycerins verestert und die Esterreste von gesättigten, ungesättigten, geradkettigen oder verzweigten Carbonsäuren mit 6 bis 22 C-Atomen abgeleitet sind, worin an eine oder zwei der nicht veresterten Hydroxylgruppen des Monoglycerins über eine Etherbrücke ein Polyalkylenglykolrest gebunden ist, der aus Ethylenoxid- und/oder Propylenoxideinheiten aufgebaut ist, und wobei die Derivate zwischen 1 und 40 Mol Alkylenoxid pro 1 Mol Monoglycerin enthalten.

**14.** Zusammensetzung nach einem oder mehreren der Ansprüche 1 bis 5, **dadurch gekennzeichnet, dass** sie mindestens eine Verbindung der Formel (1), worin jedoch n 1 bedeutet und die Summe von x, y und z 1 bis 40 beträgt, als Lösungsmittel enthält.

**15.** Verwendung einer Zusammensetzung nach einem oder mehreren der Ansprüche 1 bis 14 als Verdicker in tensidhaltigen Systemen.

**16.** Verwendung einer Zusammensetzung nach einem oder mehreren der Ansprüche 1 bis 14 zur Herstellung eines kosmetischen, dermatologischen oder pharmazeutischen Mittels.

**17.** Kosmetisches, dermatologisches oder pharmazeutisches Mittel enthaltend eine Zusammensetzung nach einem oder mehreren der Ansprüche 1 bis 14.

**18.** Mittel nach Anspruch 17, **dadurch gekennzeichnet, dass** es sich um ein Shampoo, Duschbad, Duschgel, Schaumbad, Gel, eine Lotion, Creme oder Salbe handelt.